Europäisches Patentamt

European Patent Office

Office européen des brevets

(11) Publication number: **0 063 875**
A2

## EUROPEAN PATENT APPLICATION

(21) Application number: 82301761.1

(51) Int. Cl.³: **A 61 K 7/48, A 45 D 44/22**

(22) Date of filing: **02.04.82**

(30) Priority: **16.04.81 JP 55113/81**

(71) Applicant: **MAX FACTOR & CO, 1655 North McCadden Place, Holywood California 90028 (US)**

(43) Date of publication of application: **03.11.82 Bulletin 82/44**

(72) Inventor: **Okamura, Sadao, 370, Yachimata-machi, Inbagun Chiba-ken (JP)**

(84) Designated Contracting States: **AT BE CH DE FR GB IT LI LU NL SE**

(74) Representative: **Harrison, David Christopher et al, MEWBURN ELLIS & CO 2/3 Cursitor Street, London EC4A 1BQ (GB)**

(54) **Cosmetic face mask.**

(57) A cosmetic face mask for use in performing cosmetic face pack treatments is provided which includes a sheet of material carrying liquid ingredients for the desired treatment. The sheet of material is sized and shaped to cover the entire face of a user and includes openings that correspond to the user's mouth, nose and eyes.

-1-

COSMETIC FACE MASK

This invention relates to a cosmetic product.
More particularly, this invention relates to face packs.

Face packs are cosmetic products which have
been used for many years. The prior art face packs can
be classified into the following three types by their
form of their ingredients: (1) jelly type face packs, (2)
paste type face packs and (3) powder type face packs.
The prior art face packs are used by wetting a powder or
film-forming material primarily made of water soluble
polymer, applying the wetted material to skin surfaces in
a suitable thickness and drying it for a certain period
of time. During the drying time, the layer of horny
substance in the skin, or stratum corneum, becomes wet and
soft by the moisture coming from the face packs and from
beneath the skin. In addition, the skin is contracted
slightly. After drying, the temperature of the skin is
increased temporarily to quicken the blood stream. Moreover, the face packs have absorption effect and when they are peeled off, they carry away dirt on the skin to make it clean. Therefore, the face packs have good effects on the skin, including quickening of the blood stream and cleaning.

The jelly type face packs are transparent or translucent jelly comprising polyvinyl alcohol, hydroxyethyl cellulos/carboxyvinyl polymer or the like. After being applied to the face, the resulting film is peeled off, wiped off or washed off.

The paste type face packs are opaque paste comprising vinyl acetate resin, montmorillonite, or the like. Like the jelly type face packs, they are first applied to the face and, after drying, the resulting film is peeled off, wiped off, or washed off.

The powdery face packs are made of kaolin, talc, magnesium oxide and the like which are mixed with water homogenously, and applied to the face and, after drying, washed away.

However, the prior art fluid face packs have many disadvantages in their production, storage and method of use. One disadvantage with the method of use is that users have to take out the face pack ingredients from a tube or bottle onto the tip of fingers or the palm of the hand and apply the pack to the face in a uniform thickness avoiding the eyes, nostrils and mouth. Coating the face with the fluid face packs in a uniform thickness avoiding the eyes, nostrils and mouth is not as easy as it first looks. Also, the

-3-

0063875

users are apt to grudge the trouble of washing away or wiping off the face pack remaining on the tip of fingers or on the palm of the hand. Moreover, the users cannot speak and laugh but must keep still, breathing quietly, for about 15 minutes until a film of the face pack is entirely formed on the face. Otherwise, the resulting film has many wrinkles and has little packing effect. After packing, the film on the face must be peeled off by hand or wiped off by a wet towel or washed away with tepid water. It is almost impossible to remove all of the film from the face at one time and some unpeeled pieces of the film are left on the face. This is one of the largest discomforts to the user, and it is caused by applying the face packs on the face in an uneven thickness and by the low strength of the film formed on the face. Wiping off the film with a wet towel or washing the face with tepid water is also cumbersome and users have long desired the development of a new type of face pack requiring no labor.

One of the disadvantages of the conventional fluid face packs with regard to production and storage is that it is relatively difficult to modify their viscosity. Another disadvantage is that because of their high viscosity, it is also difficult to pack them into a squeeze tube or a bottle with screw closure. The viscosity of the face pack is clearly related to not only ease of application but also to the stability of the pack, so the viscosity must be adjusted to a proper range and its change with time must be carefully checked. In addition, it is necessary to carefully study such factors as transparency (turbidity), degree of separation between oil and water, roping quality, ease of application, film strength, physical properties of film, drying time, degree of skin contraction, ease of peeling, degree of moisturization of the skin after peeling of the film, degree of dirt removed and ease of washing out of washed-type face packs.

Therefore, it has been strongly desired to develop face packs which are easy to use without requiring much labor and which can be prepared with ease.

It is an object of the present invention to provide an entirely new type of face pack which has none of the disadvantages that accompany the production, storage and use of the prior art face packs.

This and other objects and advantages of the present invention will appear by referring to the accompanying drawings and as the following description proceeds.

FIG. 1 is a plan view of a cosmetic face mask embodying the present invention;

FIG. 2 is a plan view of the cosmetic face mask shown in FIG. 1 and illustrating a further feature of the present invention;

FIG. 3 is a plan view of another embodiment of the present invention; and

FIG. 4 is a plan view of a cosmetic face mask illustrating a still further embodiment of the present invention.

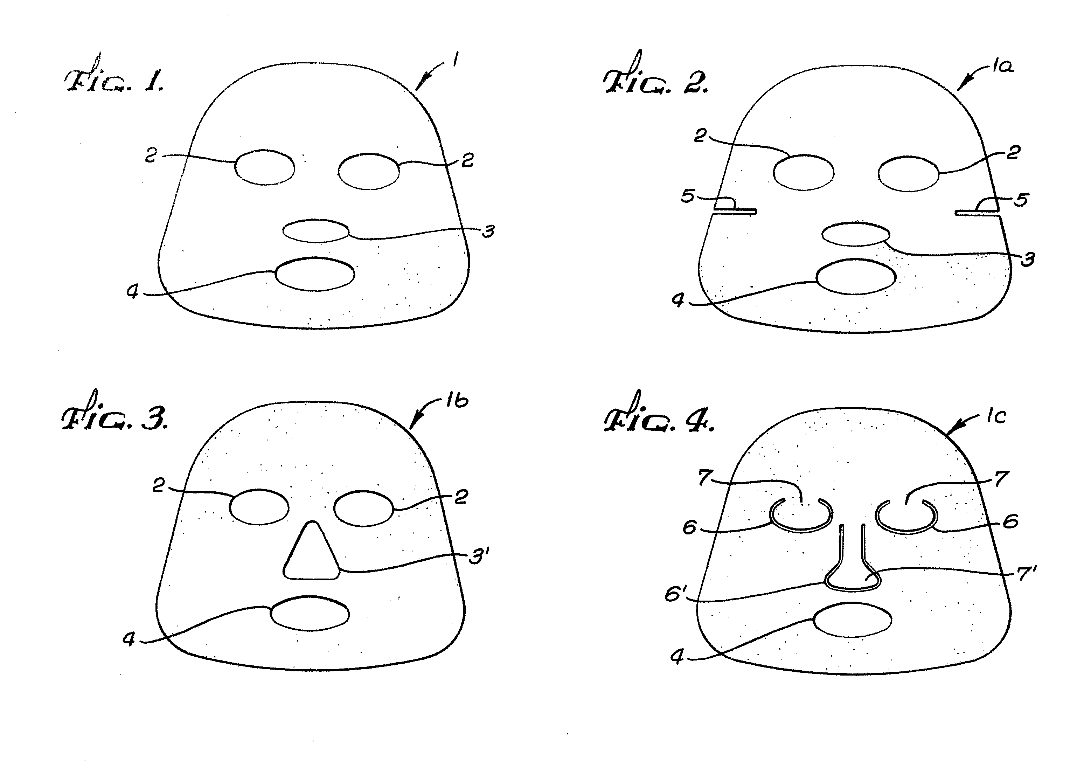

Briefly, as illustrated in exemplary Fig. 1, the present invention provides a face mask member 1 made of nonwoven fabric, paper or textile fabric soaked with liquid ingredients· for pack treatment. Nonwoven fabric, paper and textile fabric are cut into a mask so as· to substantially fit the shape of the human face, namely, a mask large enough to cover the entire face. Then, openings 2, 3 and 4 which correspond to the mouth, nose and eyes respectively are formed by cutting or blanking the mask.

The material which can be used for preparing the face mask member of the present invention includes nonwoven fabric, paper and textile fabric. As the paper, Japanese paper and machine-made paper may be used. The textile fabric is made from cotton yarn or synthetic resin yarn or a mixture thereof.

The thickness of nonwoven fabric, paper and textile fabric which are used for making the face mask members of the present invention is not critical. Generally, their thickness is held to a minimum to provide good adhesion to the face, but they must at least have a certain degree of mechanical strength and stretchability so as to avoid breaking during use.

As shown in Fig. 2, one may optionally cut the blank into a face mask member 1a with at least one slit 5 at a portion which corresponds to the cheekbone, around the jaws or other areas in which deep wrinkles may be formed in the face mask member put on those areas. The slit will serve to provide firmer adhesion of the face mask member to the face.

The size and shape of openings in the member which correspond to eyes, nose and mouth are also not critical to the present invention. For example, as shown in Figs. 1 and 2, the opening 3 corresponding to the nose may be of a size which does not prevent breathing by closing the nostrils. Alternatively, as shown in Fig. 3, the opening 3´ corresponding to the nose may have such a size and shape that all or most part of the nose is exposed. Thus, the term "opening corresponding to the nose" used throughout the specification means both the opening of a size through which only the nostril is exposed and the opening of a size through which all or most of the nose is exposed.

In order to obtain satisfactory packing effect on the eyelids and/or the bridge and the side slant surfaces of the nose without forming deep wrinkles in the face mask member, portions corresponding to the eyes and nose can be partially cut, as indicated by numerals 6 and 6´ in Fig. 4, so as to form flaps 7 and 7´ on the face mask member. The user may optionally remove the flaps from the face mask member. Thus, the term "cut-formed openings" used throughout the specification include both openings having flaps and those having no flaps. Conventional means such as scissors, knife and the like may be used for cutting or blanking the member.

0063875

Liquid ingredients which have been used in the prior art fluid face packs can be used as the liquid ingredients for soaking the face mask member of the present invention. For example, the prior art face packs commonly contain humectant such as propylene glycol, polyethylene glycol, dl-pyrrolidonecarboxylic acid salts, sorbitol and glycerine, ethanol, perfume, preservative, purified water, oily components, and optionally vitamins, amino acids or other drugs. These additives can be used in this invention individually or in combination.

The amount of liquid ingredients soaked in the face mask member is not critical to the present invention. The only requirement is to soak the face mask member with a minimum amount of liquid ingredients necessary to obtain the desired packing effect.

The face mask member of the present invention soaked with liquid ingredients can be sold in a bag made from synthetic resin or aluminum foil.

The users can perform pack treatment simply by taking the face mask member out of the synthetic resin or aluminum foil bag and unfolding and putting it on the face. The period for the pack treatment ranges from about 5 to 7 minutes. After that period, the face mask member can be removed from the face with great ease. Unlike the conventional product, the face mask member of the present invention can be peeled off without leaving any flakes on the face. In addition, the face mask member of the present invention can be usually removed without wiping or washing.

The user who wants a hot pack treatment with the prior art fluid face packs has to use an apparatus such as infrared heating unit, whereas according to the present invention, she can achieve the same effect without using a special apparatus but by heating the face mask member in hot water as it is packed in an aluminum foil bag and putting it on the face. One of the effects of the hot pack treatment is that it opens sweat glands and allows effete matter or dirt to be discharged to the outside of the skin. In addition, in the summer season in which a makeup with refreshing effect is desired, the user can enjoy pack treatment with high refreshing effect by using the face mask member of the present invention that has been cooled in a refrigerator.

Therefore, the face mask member of the present invention has a wide range of applications in comparison with the prior art fluid face packs and its packing effect is equal or superior to that of the prior art fluid face packs.

CLAIMS:

1.    A cosmetic face mask comprising a sheet of material carrying liquid ingredients for a face pack treatment, said sheet being sized and shaped to cover the entire face of a user and having openings that correspond to the mouth, nose and eyes of the user.

2.    A cosmetic face mask as set forth in claim 1, wherein said sheet is comprised of an absorbent material.

3.    A cosmetic face mask as set forth in claim 1, wherein said sheet of material is comprised of a nonwoven fabric.

4.    A cosmetic face mask as set forth in claim 1, wherein said sheet of material is comprised of paper.

5.    A cosmetic face mask as set forth in claim 1, wherein said sheet of material is comprised of textile fabric.

6.    A cosmetic face mask as set forth in claim 1, further including at least one slit to permit shaping of said mask to fit the contour of a user's face.

7.    A cosmetic face mask as set forth in claim 6, including two of said slits, located at a portion of said mask which corresponds to the cheekbones of the user.

0063875

8.    A cosmetic face mask as set forth in claim 1, wherein said openings corresponding to the eyes of said user are covered by a movable flap.

9.    A cosmetic face mask as set forth in claim 1, wherein said opening corresponding to the nose of said user is covered by a movable flap.

Fig. 1.

Fig. 2.

Fig. 3.

Fig. 4.

1/1

0063875